# EUROPEAN PATENT APPLICATION

(11) **EP 3 354 375 A1**
(43) Date of publication of application: **01.08.2018**
(21) Application number: 17305087.3
(22) Date of filing: 27.01.2017
(51) Int. Cl.: B22F 1/00, B22F 1/02, B22F 9/24, A61K 49/18

(54) **NANOMATERIAL AND METHOD OF PRODUCTION OF A NANOMATERIAL FOR MEDICAL APPLICATIONS, SUCH AS MRI OR SERS**

(71) Applicant: UNIVERSITE PARIS NORD, 93430 Villetaneuse (FR); Centre National de la Recherche Scientifique, 75794 Paris Cedex 16 (FR)
(72) Inventor: SPADAVECCHIA, Jolanda, 75011 PARIS (FR)
(74) Representative: Dejade & Biset

(57) **Abstract**

A method for producing nanomaterial product which comprises at least one nanoparticle Gold-metal-Polymer, the polymer comprising at least one biopolymer, the atoms of metal being linked with the atom of Gold, the metal being chosen among: Gd, Co, Eu, Tb, Ce, Mn, Fe, Zn, Ag, the method being realized in an aqueous solvent, without reactive or stabilizer agent, and presenting a step of reducing: tetrachloroauric acid (HAuCl₄) and metal ions, in the presence of the biopolymer, the biopolymer being used as a stabilizer agent.

## Description

### FIELD OF THE INVENTION

The invention generally relates to nanomaterials and the use of nanomaterials for analysis, diagnosis, or medical treatments.

By "medical treatment" is meant here, inter alia, targeting systems to tumors and an add-on improving efficacy or reducing adverse effect of physical treatment against cancer or vascular defects.

By "diagnosis" is meant here, inter alia, the use of medical imaging in the process of determining the nature and circumstances of a status of an organ, and more particularly Magnetic Resonance Imaging (MRI).

By "nanomaterials" is designated here nanoparticles or materials comprising nanoparticles (e.g. liquid suspension), nanoparticles being structures having a size from a few nanometers to hundreds of nanometers.

By "analysis" is designated here, inter alia, Surface Enhanced Raman Spectroscopy (SERS), the nanomaterials of the present invention being suitable for use during preparation of substrate for SERS.

By the expression "medical treatment" is also designated here the use of nanomaterials for cancer or vascular treatment, for example in the delivery of drugs for cancer treatment, by passively and actively targeting to tumor cells and also in enhancing effect of physical treatment (such as phototherapy, thermotherapy). Specific targeting of cancer cells for imaging and treatment is thought to be a key component of cancer management in the near future. Molecular imaging with targeted MRI contrast agent had emerged as one of the promising diagnostic approaches offering high resolution depiction of pathological anatomy and detection of associated disease biomarkers.

The invention relates, more particularly, on the production of nanomaterials that can be used as paramagnetic contrast agent for MRI or as add-on improving efficacy or reducing adverse effect of physical treatment, such as thermic treatment, radio treatment, photo-treatment against cancer or vascular defects.

### BACKGROUND OF THE INVENTION

Nanoparticles are used in various imaging techniques such as MRI (e.g. Padmanabhan et al, Nanoparticles in practice for molecular-imaging applications: an overview, Acta Biomaterialia, 41, 2016, pp. 1-16).

Magnetic resonance imaging (MRI) is a non-invasive imaging technique, extensively used in the clinic, which allows for high definition pictures of a whole-organism to be obtained over extended periods of time. In medical MRI, radiologists are most interested in looking at the NMR signal from water and fat, the major hydrogen containing components of the human body. MRI contrast depends on endogenous differences in water content (proton density) and relaxation time (T1, T2) in the tissue of interest.

It can be demonstrated that the dipolar interaction between free or unpaired electrons and water molecules induces a dramatic decrease in T1 and T2. Among the metals possessing unpaired electrons, gadolinium (Gd³⁺), manganese (Mn²⁺) and iron (Fe³⁺) appear the most powerful paramagnetic compounds, with 7, 5 unpaired electrons respectively.

Several materials have been developed to enhance the image contrast and diagnostic accuracy of MRI, these materials, named contrast agents, being of two different types: superparamagnectic compounds and paramagnetic compounds. Superparamagnectic contrast agents, also named T2 agents or negative contrast agents, are usually constructed with iron oxide. Paramagnetic contrast agents, also named T1 or positive contrast agents, are using paramagnetic metal ions having unpaired electrons in their outer shell (transition and lanthanide metals). The two main and widely used compounds of this class are gadolinium (Gd³⁺) or manganese (Mn²⁺).

Because of the toxicity, even at low concentrations, of free gadolinium, it is usually bound to a chelate (a low-molecular weight organic molecule e.g. DTPA5 diethylene triamine pentaacetic acid). Both gadolinium and the ligands alone cannot be used because of their toxicity. Commonly used contrast agents based on gadolinium chelates have been sold under the following trademarks:
- Magnevist (gadopentetate dimeglumine, DTPA-Gd), a linear ionic complex of the gadolinium ion (Gd³⁺) and polyaminocarboxylate ligands, more precisely N-methylglucamine salt of the gadolinium complex of diethylenetriamine pentaacetic acid;
- Omniscan (gadodiamide, DTPA-BMS-Gd) gadolinium neutral linear complex of diethylenetriamine pentaacetic acid bismethylamide;
- Dotarem (gadoterate meglumine, DOTA-Gd) is a macrocyclic ionic complex, having empirical formula of C₂₃H₄₂O₁₃N₅Gd (anhydrous basis), CAS Registry No. 92943-93-6;
- ProHance (gadoteridol, HP-DO3A-Gd) is a neutral macrocylic gadolinium complex of 10-(2-hydroxy-propyl)-1,4,7,10-tetraazacyclododecane 1,4,7-triacetic acid, empirical formula of C₁₇H₂₉N₄O₇Gd;
and also Gadovist (gadobutrol), Optimark (gadoversetamide), Multihance (gadobenate dimeglumine), Primovist, Eovist (gadoxetate), Vasovist.

The Gd (III) chelate for clinical applications, has been divided into two major groups of cyclic (the macrocyclic ligands, e.g. DOTA and DO3A) and acrylic (the acryclic ligands, e.g. DTPA and DTPA-BMA).

Other gadolinium based magnetic resonance contrast agents for molecular imaging have been proposed, e.g. dendrimer, hybrid nanoparticles, and targeted contrast agents, and also:
- macromolecular complexes, formed by attaching Gd (III) chelates to macromolecules, slowing down the rotational motion of the complexes, thus increasing relaxivities (for example Gd³⁺-hexanedione NPs);
- biodegradable macromolecular, for excreting rapidly as low molecular-weight agents (e.g. polydisulfide Gd(III));
- liposomal particles Gd(III) complexes including Gd-DTPA, Gd(DTPA-BMA) and Gd-DOTA have been encapsulated in the core of liposomes to prepare nano-scaled MRI contrast agents.

Nanoparticles containing gadolinium used as contrast agent are proposed in prior art, the nanoparticles being obtained by encapsulating gadolinium in the core space of the nanoparticles; carrying gadolinium on the exterior of the nanoparticle via self-assembling technologies; or carrying gadolinium in the exterior of the nanoparticle via chemical conjugation.

WO 2011/113616 discloses a nanoparticle comprising a micelle formed by an amphiphilic block-copolymer and encapsulating a gadolinium complex, the nanoparticles being used as contrast agent for MRI for breast cancer early detection.

US 2016/0051707 discloses a method for producing a metal oxide nanoparticle-based T1-T2 dual-mode MRI contrast agent that has a core of T1 contrast material and a porous shell of T2 contrast material on the core, comprising the following steps: synthesizing metal oxide nanoparticles of T1 contrast material under inert gas environment; forming an epitaxial layer of metal oxide of T2 contrast material on the surface of metal oxide nanoparticles of T1 contrast material under inert gas environment; maintaining the formation of the layer of metal oxide of T2 contrast material under dry air environment to form multilayer nanoparticles having a core and porous shell structure; and coating multilayer nanoparticles with a biocompatible polymer selected from the group consisting of biopolymers such as chitosan, elastin, hyaluronic acid, alginate, gelatin, collagen, and cellulose; and synthetic polymers such as polyethylene glycol (PEG).

Chen et al (Gadolinium conjugated PLA-PEG nanoparticles as liver targeted molecular MRI contrast agent, Journal of drug targeting, 2011, pp. 657-665) discloses spherical nanoparticle MRI contrast agent targeted to liver developed by conjugation of gadolinium chelate groups onto the biocompatible poly(I-lactide)-block-poly(ethylene glycol) (PLA-PEG) nanoparticles. PLA-PEG conjugated with diethylenetriaminopentaacetic acid (DTPA) was used to formulate PLA-PEG-DTPA nanoparticles by solvent diffusion method, and then gadolinium was loaded onto the nanoparticles by chelated with the unfolding DTPA on the surface of the PLA-PEG-DTPA nanoparticles.

One drawback of the process discloses by Chen et al is that one polymer molecule could only be link to one chelate molecule, the amount of the surface Gd being thus limited. To overcome this drawback, Liu et al (Gadolinium-loaded polymeric nanoparticles modified with Anti-VEGF as multifunctional MRI contrast agents for the diagnosis of liver cancer, Biomaterials, 2011, pp. 5167-5176*),* discloses a multifunctional polymeric nanoparticle contrast agent (Anti-VEGF PLA-PEG-PLL-Gd NP) simultaneously modified with Gadolinium-diethylenetriamine pentaacetic acid (Gd-DTPA) and anti-vascular endothelial growth factor (VEGF) antibody to deliver Gd-DTPA to the tumor area and achieve the early diagnosis of hepatocellular carcinoma (HCC).

Park et al (Gold nanoparticles functionalized by gadolinium DTPA conjugate of cysteine as a multimodal bioimaging agent, Bioorganic & Medicinal Chemistry Letters 20, 2010, pp. 2287-2291), discloses the synthesis of gold nanoparticles coated with Gd-chelate (Au@GdL), where L is a conjugate of DTPA and cysteine, these particles being obtained by the replacement of citrate from the gold nanoparticle surfaces with gadolinium chelate (GdL).

Nicholls et al (DNA-gadolinium-gold nanoparticles for in vivo T1 MR imaging of transplanted human neural stem cells, Biomaterials 77, 2016, pp. 291-306*)* discloses a gold nanoparticle conjugate that is functionalized with modified deoxythymidine oligonucleotides bearing Gd (III) chelates and a red fluorescent Cy3 moiety to visualize in vivo transplanted human neural stem cells.

Nasiruzzaman et al (Gold nanoparticles coated with Gd-chelate as a potential CT/MRI bimodal contrast agent, Bull Korean Chem Soc, 2010, pp. 1177-1181) discloses the synthesis of gold nanoparticles coated by Gd-chelate (GdL@Au), where L is a conjugate of DTPA and 4-aminothiophenol. These particles are obtained by the replacement of citrate from the gold nanoparticle surfaces with gadolinium chelate (GdL).

Darras et al (Chitosan modified with gadolinium diethylenetriaminepentaacetic acid for magnetic resonance imaging of DNA/chitosane nanoparticles, Carbohydrate polymers 80, 2010 pp. 1137-1146) discloses production of spherical nanoparticles with diameters in the range of 30-150 nm, using low molecular weight chitosan and covalent linkage of diethylenetriaminepentaacetic acid (DTPA) to chitosan amine groups, Gd being located preferentially in a 2-5 nm wide area surrounding the nanoparticles.

Lopez-Neira et al (Surface Enhanced Raman Scattering of Amino Acids Assisted by Gold Nanoparticles and Gd3+ Ions, The journal of physical chemistry, 2015, pp. 4127-4135) discloses fixation of Gd³⁺ on L-tyrosine coated gold nanoparticles.

Wen et al (Multifunctional dendrimer-entrapped gold nanoparticles for dual mode CT/MR imaging applications, Biomaterials Volume: 34 Issue 5, 2013, pp. 1570-1580*)* discloses the synthesis of gadolinium-loaded dendrimer-entrapped gold nanoparticles (Gd-Au DENPs) for dual mode computed tomography/magnetic resonance imaging applications. In this prior art document, amine-terminated generation five poly(amidoamine) dendrimers modified with gadolinium chelator and polyethylene glycol (PEG) monomethyl ether were used as templates to synthesize gold nanoparticles (AuNPs). Followed by sequential chelation of Gd(III) and acetylation of the remaining dendrimer terminal amine groups, multifunctional Gd-Au DENPs were formed.

One manganese Mn II chelate was available for MRI as an hepatobiliary compound used for the diagnosis of liver and pancreas lesions: mangafodipir trisodium (Mn-DPDP, Teslascan®). This was a contrast agent that consists of manganese and an organic ligand, fodipir (dipyridoxyl diphosphate, DPDP). Thus product has been described in 1989 (Rocklage et al, Manganese(II) N,N'-dipyridoxylethylenediamine-N, N'-diacetate 5,5'-bis(phosphate). Synthesis and characterization of a paramagnetic chelate for magnetic resonance imaging enhancement, Inorganic Chemistry 1989, 28, pp. 477-485)*.* This Mn II contrast agent was approved by the FDA in 1997.

### PROBLEMS TO BE SOLVED

One of the problems associated with the use of Gd(III) or Mn(II) is toxicity. Another problem is that the production of nanoparticles is long and complicated.

The gadolinium metal ion is toxic in vivo, gadolinium having an ionic radius similar to the calcium ion and interfering with numerous metal dependent enzymatic systems, and extremely interfering with calcium channels and proteins binding sites.

Thus, gadolinium III ions cannot be administrated directly. Free gadolinium ions accumulate in the liver, spleen, kidney and bones.

To reduce the side effects of toxic ions and prevent tissue interaction, Gd (III) ions are combined with chelating ligands. But Toxic Gd(III) ions may still be released of some chelates via transmetallation with other metal ions such as Zn²⁺, Ca²⁺ and Cu²⁺ in the body and protonation of the ligands in the pH low which may cause the separation of scheelite within the body.

Nephrogenic fibrosing dermopathy (NFD) is an idiopathic disorder in kidney patients. In most patients with NFD, dialysis for kidney failure occurs. Gd-containing contrast agents in patients with normal kidney function are rapidly excreted from the kidney with a half-life of about two hours. However, in patients with chronic renal failure Gd-containing contrast agents have a long half-life, may be greater than 120 to 30 hours. The combination of metabolic acidosis and insufficient clearance of Gd-containing agent is present in renal failure.

Toxicity is also present for manganese contrast agent. Mangafodipir trisodium Mn-DPDP readily dissociates to yield free manganese ions. This in vivo instability of the chelate rose concerns about potential toxicity. Free manganese, in chronic exposure, causes a parkinsonism-like syndrome due to accumulation in the brain. Furthermore, a significant neurological risk is associated with manganese intoxication in subjects with liver dysfunction/hepatic encephalopathy whose ability to eliminate manganese is reduced. It can also have a depressive action on heart function.

Mn-DPDP was withdrawn from the US market in October 2003 and the European market in July 2010.

To try to overcome the drawbacks of Mn-DPDP, attempts are made to provide manganese based MRI contrast agents, see e.g. US9198988 (Kent State University).

There is still a need inter alia for nanoparticles that can be used as high-performance contrast agents with reduced toxicity. There is still a need for nanoparticles that can be used as add-on improving efficacy or reducing adverse effect of physical treatment, such as thermic treatment, radio treatment, photo-treatment against cancer or vascular defects, these nanoparticles having a reduced toxicity.

### SUMMARY OF THE INVENTION

One object of the invention is a method for producing nanomaterial product which comprises at least one nanoparticle gold-metal-polymer, the polymer comprising at least one biopolymer, the metal being chosen among: Gd, Co, Eu, Tb, Ce, Mn, Fe, Zn, Ag, the method being realized in an aqueous solvent, the method presenting a step of reducing gold ions and metal ions, in the presence of the biopolymer, the biopolymer being used as a stabilizer agent.

By biopolymer is meant here a polymer that can be produced by a living organism which is natural.

Advantageously, the method is realized without chemical surfactant and without chemical linker. The method of the invention does not need to use chemical surfactant and synthetic chemical linker. In other terms the method of invention does not need to use reactive or stabilizer agent used in the methods of prior art.

The method of the invention presents very fast steps in comparison with the methods of the state of the art, and is simple to realize, in order to obtain the nanoparticles.

Another object of the invention is such a method, the reduction being realized in a presence of a biopolymer mixture which comprises at least two biopolymers, one of the biopolymer comprising COOH group.

Another object of the invention is such a method comprising the successive steps: (Ai) introduction of metal ions in the aqueous solvent which comprises AuCl₄⁻, for realizing complexation between metal ions and gold ions conducted to form gold-metal complex, during a first period; (Aii) stacking, by mixing said gold-metal complex with a first biopolymer introduced in the aqueous solvent, for obtaining a gold-metal-polymer complex, during a second period; (Aiii) reduction of the metal ions and reduction of the gold ion of the gold-metal-polymer complex, by introducing the reducing agent, during a third period.

Advantageously, step Ai of introduction of metal ions in the aqueous solvent which comprises AuCl₄⁻ is done under stirring.

After centrifugation, a nanomaterial product is obtained, having an inner core with gold atom and metal complex, and a polymer outer shell.

Another object of the invention is such a method comprising the successive steps: (Bi) staking by mixing tetrachloroauric acid HAuCl₄ with a first biopolymer for obtaining a gold polymer complex, during a first period, (Bii) complexation by addition of metal ions to the gold polymer complex for obtaining a gold-metal-polymer complex, during a second period, (Biii) reduction of metal ions and reduction of gold ions of the gold-metal-polymer complex, by introducing a reducing agent, during a third period.

Advantageously, step Bii of complexation is done with stirring.

After centrifugation, a nanomaterial product is obtained, having an inner core with gold atom and metal complex, and a biopolymer outer shell.

Another object of the invention is such a method comprising a step of adding a second biopolymer: between the step (Aii) and the step (Aiii), or between the step (Bi) and the step (Bii).

Advantageously, the steps of the method are conducted at room temperature.

Advantageously, the first period, the second period and the third period last each less than fifteen minutes.

Advantageously, the first biopolymer and the second biopolymer are chosen among collagen, chitosan, polyethylene glycol diacide, alginate.

Advantageously, one of the biopolymer is chosen among: collagen, polyethylene glycol diacide.

Advantageously, the metal is gadolinium and the first biopolymer is collagen.

Another object of the invention is a nanomaterial product synthetized as described above, with at least one nanoparticle gold-metal-polymer, the metal being chosen among: Gd, Co, Eu, Tb, Ce, Mn, Fe, Zn, Ag, the polymer being at least one biopolymer comprising COOH group.

Advantageously, the nanoparticle comprises at least two biopolymers, one of the biopolymer comprising COOH group.

Another object of the invention is a nanomaterial product synthetized as described above, with at least one nanoparticle gold-metal-polymer, the metal being chosen among Gd, Co, Eu, Tb, Ce, Mn, Fe, Zn, Ag, the polymer being at least one biopolymer, ionic bonds being formed between the atom of gold and the atoms of metal.

Another object of the invention is such a nanomaterial product, the nanoparticle comprising an inner core with at least one atom of gold and atoms of metal surrounding the atom of gold, and an external shell with the biopolymer(s).

According to some embodiments, the nanoparticle comprises an inner core with one atom of gold, and an external shell with atoms of metal and the biopolymer(s).

Advantageously, the external shell of the nanoproduct has a diameter comprised between 50 and 200 nm.

Advantageously, the external shell of the nanoparticle has a diameter comprised between 5 and 50 nm. Here, the diameter comprises the biopolymer with the complex Gd-Au of the nanoparticle.

In some embodiments, the metal is Gd, and the biopolymer or one of the biopolymers is collagen.

In some embodiments, the metal is Gd, and the biopolymers are collagen and PEG.

In some embodiments, the metal is Gd, and the biopolymers are PEG and alginate.

In some embodiments, the metal is Gd, and the biopolymers are PEG and chitosane.

Another object of the invention is the use of the nanomaterial product as presented above, as a substrate for Enhanced Raman Spectroscopy (e.g. SERS), or as a contrast agent for Magnetic Resonance Imaging (MRI).

Another object of the invention is the use of the nanomaterial product as presented above, as a therapeutic agent, a radio sensitive agent, a thermo-therapy agent, a radioactive agent, or a phototherapy agent.

The present invention is directed to address one or more of the problems set forth above. The following presents a simplified summary of the invention in order to provide a basic understanding of some aspects of the invention. This summary is not an exhaustive overview of the invention. It is not intended to identify key of critical elements of the invention or to delineate the scope of the invention. Its sole purpose is to present some concepts in a simplified form as a prelude to the more detailed description that is discussed later.

While the invention is susceptible to various modification and alternative forms, specific embodiments thereof have been shown by way of example in the drawings. It should be understood, however, that the description herein of specific embodiments is not intended to limit the invention to the particular forms disclosed.

### BRIEF DESCRIPTION OF THE DRAWINGS

The objects, advantages and other features of the present invention will become more apparent from the following disclosure and claims. The following non-restrictive description of preferred embodiments is given for the purpose of exemplification only with reference to the accompanying drawings in which:
- Fig. 1a, 1b and 1c are views of nanomaterial according to some embodiments of the present invention;

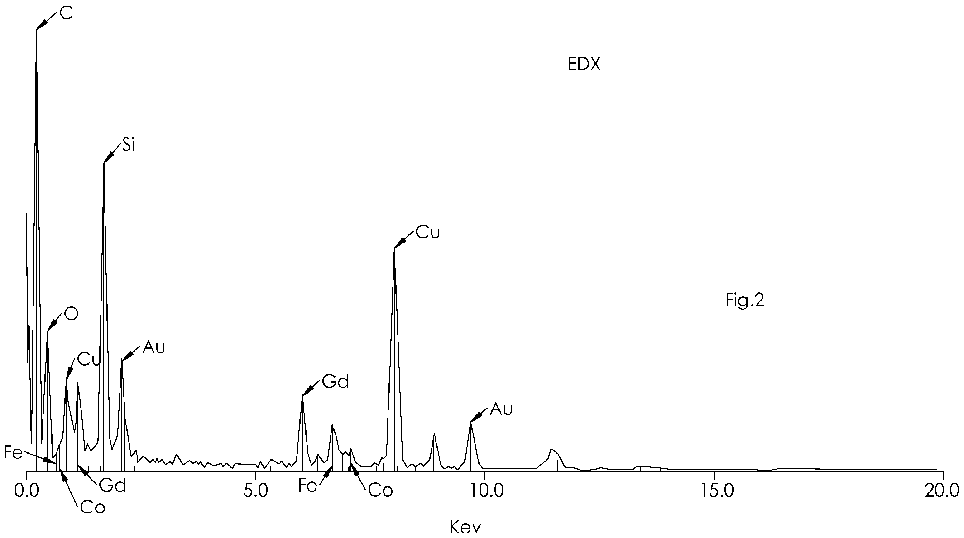

- Fig. 2 is an EDX spectrum performed on Au-Gd-polymer nanomaterial;
- Fig. 3 is a UV-VIS spectra of Au-Gd-polymer nanomaterials;
- Fig. 4 and Fig.5 are Raman spectra of Au-Gd-polymer nanomaterials;

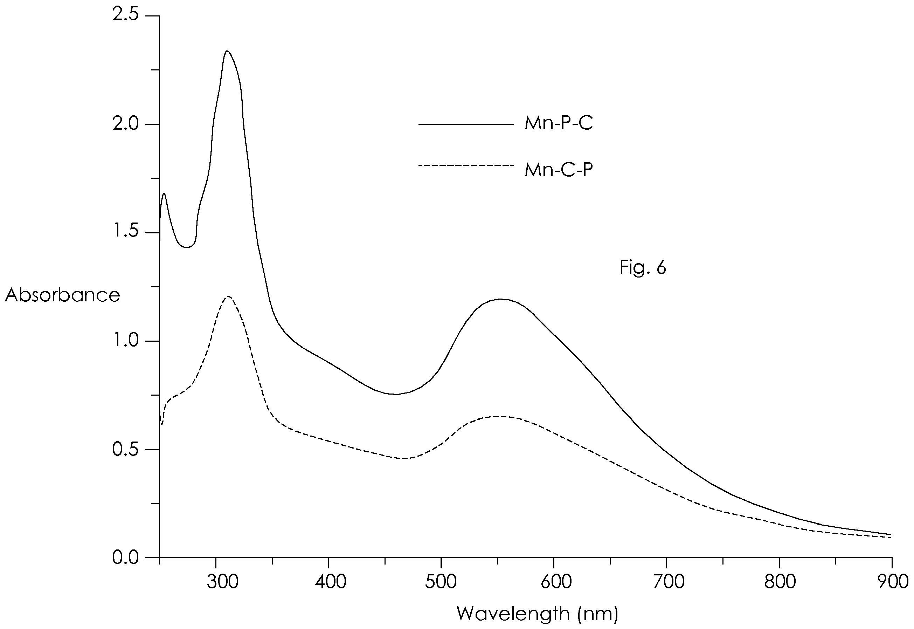

- Fig. 6 is a UV-VIS spectra of Au-Mn-polymer nanomaterials;

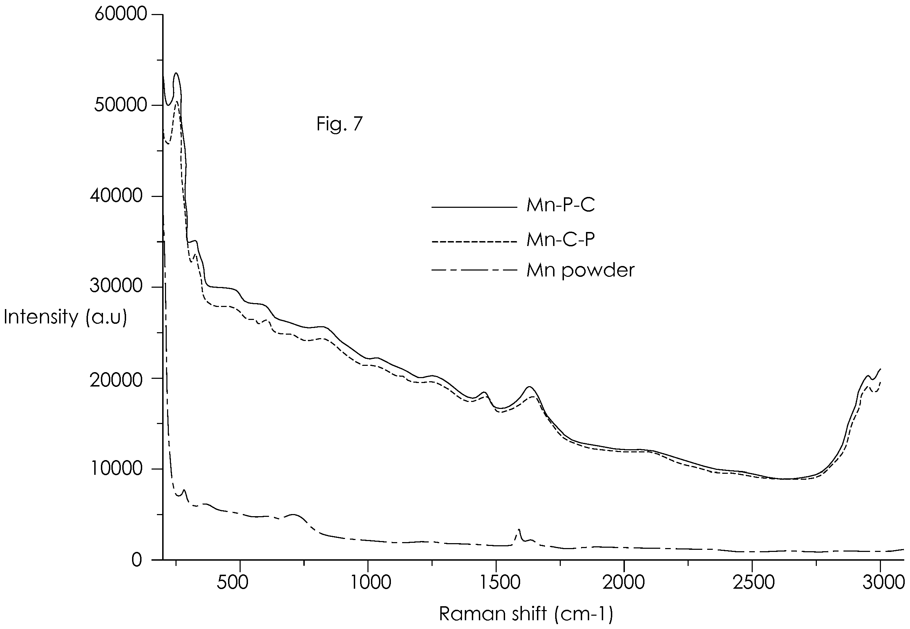

- Fig.7 are Raman spectra of Au-Mn-polymer nanomaterials;
- Fig. 8 is a schematic of proposed mechanism of GdCl₃-AuCl₄⁻reduction by complexation and particle formation in the presence of PEG, Col, ALG, CHIT as surfactants;
- Fig. 9 is a schematic of proposed mechanism of GdCl₃-AuCl₄⁻reduction and particle formation in the presence of PEG, Col, ALG, CHIT.

### DETAILED DESCRIPTION OF ILLUSTRATIVE EMBODIMENTS

### Abbreviation used

- ALG: sodium alginate;
- AuNPs: nanoparticles comprising gold;
- CHIT: chitosan (deacetylated chitin, poly(D-glucosamine));
- Col: collagen type I from calf skin;
- COSY: correlation spectroscopy;
- GdAuNPs: nanoparticles comprising gold and gadolinium;
- MnAuNPs: nanoparticles comprising gold and manganese;
- HSQC: heteronuclear single quantum coherence;
- IEFPCM polarizable continuum model using the integral equation formalism variant;
- PBS: phosphate buffer solution;
- PEG: dicarboxylic polyethylene glycol (PEG)-600;
- SEM- EDX scanning electron microscopy-energy dispersive x-ray analysis;
- TEM: transmission electron microscopy.

### Materials

Tetrachloroauric acid (HAuCl₄), gadolinium chloride hexahydrate (GdCl₃, 6H₂0), sodium borohydride (NaBH₄), PBS (pH 7,2 - 9,0), sodium chloride (NaCl), PEG, Col, CHIT, ALG were purchased from Sigma-Aldrich (Saint-Quentin Fallavier, France).

All chemicals were used as such, without further purification.

Milli Q water was used throughout the experiments.

All solvents were used without any further purification.

Experiments were carried out at room temperature, if not specified otherwise.

### Physico-chemical characterization

All the measurements were performed in triplicate, in order to validate the reproducibility of the synthetic and analytical procedures.

### UV-Vis absorption spectroscopy

Absorption spectroscopy measurements were carried out by means of a double-beam Varian Cary 500 UV-Vis spectrophotometer (Agilent, France).

Absorption spectra were recorded in the 350-900 nm spectral range in water, at AuNPs concentration equal to 10⁻⁴ M. For stability studies, Gd-AuNPs were dispersed in PBS (0, 1 M; pH 4,0 or pH 7,0), and absorption spectra collected over three months.

### TEM

TEM images were acquired with a JEOL JEM 1011 microscope (JEOL, USA) at an accelerating voltage of 100 kV.

TEM specimens were prepared after separating the PEG from the metal particles by centrifugation. Typically, 1 ml of Gd-AuNPs was centrifuged for 20 min at a speed of 14000 rpm. The upper part of the colorless solution was removed and the solid portion was re-dispersed in 1 ml of water. 2 µL of this re-dispersed particle suspension was placed on a carbon coated copper grid (manufactured by Smethurst High-Light Ltd and marketed by Agar Scientific) and dried at room temperature.

### SEM-EDX

Scanning electron microscopy (SEM) investigation was performed on an environmental SEM (ESEM, Quanta 200 FEG, FEI Company Hillsboro, OR) equipped with an (EDX) spectrometer (Genesis 2000, XMS System 60 with a Sapphire Si/Li Detector from EDAX Inc., Mahwah, NJ).

### Raman spectroscopy

The Raman experiments have been performed on an Xplora spectrometer (Horiba Scientifics-France).

The Raman spectra have been recorded using an excitation wavelength of 785 nm (laser diode) at room temperature.

For measurements in solution, a macro-objective with a focal length of 40 mm (NA = 0,18) was used in backscattering configuration. The achieved spectral resolution is close to 2 cm⁻¹.

### ¹H-NMR

Experiments were conducted on a AVANCE III spectrometer (Bruker) operating at 500 MHz with a 5 mm gradient indirect detection probe, at a probe temperature of 300 K.

64 scans were acquired to generate ID proton spectra of GdCl₃ (5 mM), PEG (1 mM) and Col, CHIT ALG products (1 mM), whereas 2048 scans were acquired for Au-Gd NP1 - Au-Gd NP5 (1 mM).

The data acquisition size was 32 K and the spectral width was 5000 Hz.

Typical 9,5 µs pulse length and relaxation delay of 2 s were used. Water signal was suppressed by applying a secondary irradiation field at the water resonance frequency (pre-saturation sequence). For each sample 500 µL aliquots were directly mixed with 100 µL Deuterium Oxide (D20) 99,96% (Eurisotop) providing an internal field-frequency lock. Samples were placed in 5 mm diameter tubes for ¹H-NMR analysis. For ¹H resonance assignment, COSY spectra were acquired for DOX with 4 K data points and 32 transients for each of the 128 increments. Moreover, 2D NMR HSQC experiments were used for ¹³C resonance assignment. Chemical shifts (δ, in ppm) were compared to the NMR solvent signal D₂0 (4,73 ppm at 300K).

### Zeta potential measurements

The zeta potential of Gd-AuNPs dispersed in water was measured using the electrophoretic mode of a Zetasizer NANOZS (Malvern Instruments Ltd, UK).

### Preparation of collagen solution (Col)

Collagen solution (Col) was diluted in PBS (pH 7,2) for 1h at room temperature.

### Preparation of Alginate solution (ALG)

10 mg of ALG was dissolved in 10 mL of PBS (pH 9,0) under ionic strength conditions (NaCl: 0.5mM) for 1h at room temperature until homogeneous solution.

### Preparation of Chitosane solution (CHIT)

10 mg of CHIT was dissolved in 10 mL of PBS (pH 9,0) under ionic strength conditions (NaCl: 0.5mM) for 1h at room temperature until homogeneous solution.

### Preparation of HAuCl₄ 6H₂O solution

16 mg of HAuCl₄ 6H₂O was dissolved in 50mL of MilliQ water at room temperature until homogenous solution.

### Preparation of GdCl₃ (Gd) solution

Solution a) 50 mg of GdCl_{3*}6H₂0 was dissolved in 50 mL of HAuCl₄ solution (9.4*10⁻⁴M) at room temperature until homogeneous solution. Solution b) 16 mg of GdCl_{3*}6H₂0 was dissolved in 50 mL of MilliQ water at room temperature until homogeneous solution.

### Preparation of MnCl₂ solution

16 mg of MnCl_{2*}6H₂0 was dissolved in 50 mL of MilliQ water at room temperature until homogeneous solution.

### Synthesis Procedures

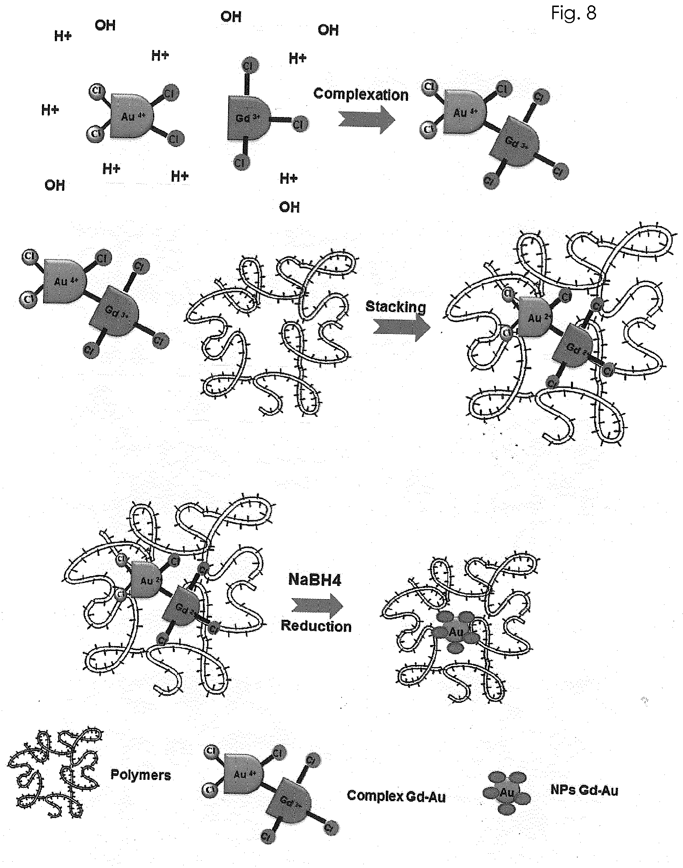

Procedures are described know with reference to the protocol represented on figure 8.

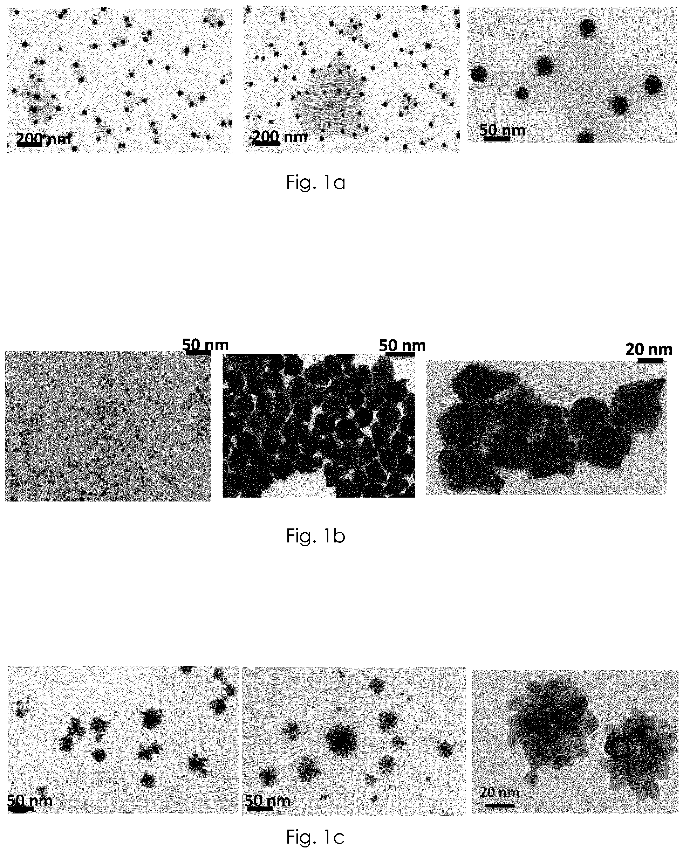

### Synthesis of Gd-AuNP1 (Gd-Au-ColNPs ), see figure 1a

Gd-Au-ColNPs were prepared by a fast steps chemical process.

1ml of Gd solution b) was mixed with 20 ml of 0.0001 M aqueous HAuCl₄ solution for 10 min. After this time, 1ml of Col solution was added for 10 min. Then 6 ml (and/or 1.2ml) of 0.01M NaBH₄ was added drop-wise to the resulting solution, followed by rapid stirring. After 1h without agitation, the solution became brune-pale. The product was centrifuged at 9000 rpm for 25 min and the pellet was recovered and purified by washing with milli Q water for three times. The entire synthesis takes less than two hours.

### Synthesis of Gd-AuNP2 (Gd-Au-PEG-ColNPs), see figure 1b

2ml of Col solution was mixed with 20 ml of 0.0001 M aqueous HAuCl₄ solution for 10 min. Then 250µl of PEG solution was mixed under stirring at room temperature. To the resulting solution 6 ml (and/or 1.2ml) of NaBH₄ (0.01 M) was added dropwise followed by rapid stirring. After 1h without agitation, the solution became red-violet. The product was centrifuged and purified at the same conditions. The entire synthesis takes less than two hours.

### Synthesis of Gd-AuNP3 (Gd-Au -PEGNPs ) see figure 1c

Gd-Au-PEGNPs were prepared under two followed protocol.
1) 1ml of Gd (solution b) was mixed with 20 ml of 0.0001 M aqueous HAuCl₄ solution for 10 min. After this time, 250 µl of PEG solution was added under stirring at room temperature. After 10 min, 3mL (and/or 1,2 ml) of NaBH₄ (0.01 M) was added drop-wise followed by rapid stirring and kept without agitation for 2h. The resulting red-rose solution was centrifuged and purified at the same conditions.
2) Gd solution a) was dissolved in 50 mL of HAuCl₄ solution (9.4*10⁻⁴ M) at room temperature until homogeneous solution. After this time, 250 µl of PEG solution was added under stirring at room temperature. After 10 min, 3mL (and/or 1,2 ml) of NaBH₄ (0.01M) was added drop-wise followed by rapid stirring and kept without agitation for 2h. The resulting red-rose solution was centrifuged and purified at the same conditions.

### Stability of Gd-AuNPs as a function of pH.

The stability of nanoparticles was detected by UV VIS. All nanoparticles were dissolved in PBS solution 0.1M at different pH (pH 1,6 7,8,12) during 18h.

### Synthesis of Mn-AuNP1 (Mn-Au-PEG-ColNPs)

5 ml of Mn solution was mixed with 20 ml of 0.0001 M aqueous HAuCl₄ solution for 10 min. After this time, 250 µl of Polyethylene glycol 600 Diacid (PEG) was added under stirring for 5 min. Then 500µl of Collagen was added under stirring at room temperature. After 10 min, 1,2 ml of NaBH₄ (0.01 M) was added drop-wise followed by rapid stirring and kept without agitation for 2h. The resulting red-rose solution was centrifuged and purified at the same conditions

### Synthesis of Mn-AuNP2 (Mn-Au-PEG-Col-ALGNPs)

5 ml of Mn solution was mixed with 20 ml of 0.0001 M aqueous HAuCl₄ solution for 10 min. After this time, 0.5ml of Collagen and 1ml of ALG solutions were added under stirring at room temperature. After 10 min, 1,2 ml of NaBH₄ (0.01M) was added drop-wise followed by rapid stirring and kept without agitation for 2h. The resulting red-rose solution was centrifuged and purified at the same conditions

### Synthesis of Mn-AuNP2 (Mn-Au-PEG-Col-CHITNPs)

5 ml of Mn solution was mixed with 20 ml of 0.0001 M aqueous HAuCl₄ solution for 10 min. After this time, 1ml of Collagen and 1ml of CHIT solutions were added under stirring at room temperature. After 10 min, 1,2 ml of NaBH₄ (0.01M) was added drop-wise followed by rapid stirring and kept without agitation for 2h. The resulting red-rose solution was centrifuged and purified at the same conditions

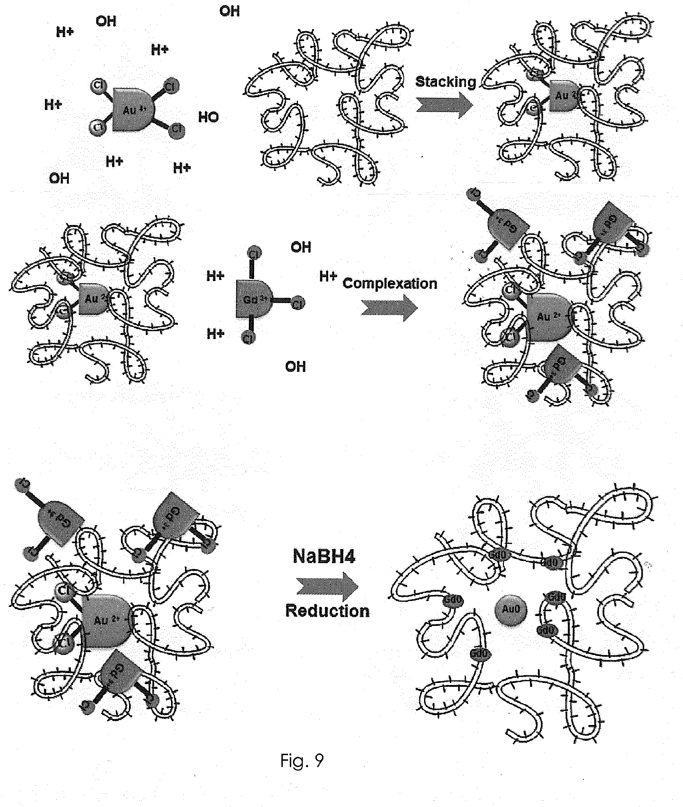

Procedures are described know with reference to the protocol represented on figure 9.

### Synthesis of Gd-AuNP1 (Gd-Au-PEG-ColNPs)

250 µl of Polyethylene glycol 600 Diacid (PEG) was mixed with 20 ml of 0.0001 M aqueous HAuCl₄ solution under stirring for 10 min. After this time, 500µl of Collagen and 1 ml of Gd (protocol b) were added under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Gd-AuNP2(Gd-Au-PEG-Col-ALGNPs)

250 µl of Polyethylene glycol 600 Diacid (PEG) was mixed with 20 ml of 0.0001 M aqueous HAuCl₄ solution under stirring for 10 min. After this time, 50µl of Collagen, 1mL of ALG solution and 1 ml of Gd solutions were added under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Gd-AuNP3(Gd-Au-PEG-Col-CHITNPs)

Gd-Au-PEG-Col-CHITNPs were prepared under same protocol of Gd-AuNP2.

250 µl of Polyethylene glycol 600 Diacid (PEG) was mixed with 20 ml of 0.0001 M aqueous HAuCl₄ solution under stirring for 10 min. After this time, 500µl of Collagen, 1mL of CHIT solution and 1 ml of Gd solutions were added under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Mn-AuNP1 (Mn-Au-PEG-ColNPs)

250 µl of Polyethylene glycol 600 Diacid (PEG) was mixed with 20 ml of 0.0001 M aqueous HAuCl₄ solution under stirring for 10 min. After this time, 500µl of Collagen, and 2 ml of Mn solutions were added under stirring at room temperature. After 10 min, 3mL of NaBH₄ (0.01M) was added drop-wise followed by rapid stirring and kept without agitation for 2h. The resulting red-rose solution was centrifuged and purified at the same conditions.

### Synthesis of Mn-AuNP2 (Mn-Au-PEG-Col-ALGNPs)

250 µl of Polyethylene glycol 600 Diacid (PEG) was mixed with 20 ml of 0.0001 M aqueous HAuCl₄ solution under stirring for 10 min. After this time, 50µl of Collagen, 1mL of ALG solution and 2 ml of Mn solutions were added under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Mn-AuNP2 (Mn-Au-PEG-Col-CHITNPs)

250 µl of Polyethylene glycol 600 Diacid (PEG) was mixed with 20 ml of 0.0001 M aqueous HAuCl₄ solution under stirring for 10 min. After this time, 500µl of Collagen, 1mL of CHIT solution and 2 ml of Mn solutions were added under stirring at room temperature. After 10 min, 3mL of NaBH₄ (0.01M) was added drop-wise followed by rapid stirring and kept without agitation for 2h. The resulting red-rose solution was centrifuged and purified at the same conditions.

### Results and discussion

Change in the surface charge (z-potential) for each Au-Gd-NP in mv (+- standard deviation) are as follows

| | |
|---|---|
| Au-Gd-NP1 | -12.8 (+-0.2) |
| Au-Gd-NP2 | -14.8 (+-2.0) |
| Au-Gd-NP3 | -13.8 (+-1.0) |

Formation mechanism of hybrid Gd-Au NPs and role of polymers as stabilizer agent.

The synthesis of Gd-AuNPs (NP1-NP3) was carried out by reducing tetraclororoauric acid (HAuCl₄) in the presence of GdCl₃ and different biopolymers (PEG, Col, ALG, CHIT) using sodium borohydride (NaBH₄) as a reducing agent by using two protocols in order to show the critical importance of order of reagents and concentrations for the shape, organization, morphology of the nanoproduct and the nanoparticles of the nanoproduct. Particles formation and growth were controlled by the amphiphilic (dual nature) character of the polymers.

In the case of the process represented on figure 8, Gd-Aupolymer modification onto the surface of AuNPs, solutions was prepared to this end. Particles formation and growth were controlled by the amphiphilic character of the polymers and includes three steps: complexation between GdCl₃ and AuCl⁴⁻ to form gold-gadolinium clusters; adsorption (stacking) of COOH-terminated polymer molecules onto Gd-Au complex; reduction of metal ions in that vicinity, growth of gadolinium-gold particles and colloidal stabilization.

In the latter case, Gd-Au complex molecules are expected to be involved in the nucleation process and may, thus, influence the final shape and size of nanoparticles.

In the case of the process represented on figure 9, the formation of golf nanoparticles from AuCl⁴⁻ can be summarized in three steps : formation of polymer mixture (i.e. PEG, Col, CHIT, ALG); initial reduction of metal ions facilitated by dicarboxylic acid-terminated PEG and/or Col to form gold clusters; adsorption (stacking) of polymers molecules on the surface of gold clusters and reduction of metals in that proximity and growth of gold particles and colloidal stabilization by molecules of polymers.

As can be seen on figures 8 and 9, depending on the process used (complexation / stacking / reduction or stacking / complexation / reduction), the metal ions (Gd, Eu, Tb, Ce, Mn, Fe, Zn, Ag) are not at the same distance to the atom of gold.

Gd (or the other metals: Co, Eu, Tb, Ce, Mn, Fe, ZN, Ag when used instead of Gd) is close to Au in the core of the nanoparticle the outer shell being essentially based on polymer (figure 8). Gd being not covalent linked with the gold atom on figure 8.

On the contrary, Gd is placed at a larger distance to Au in the outer polymer shell of the nanoparticle (figure 9), Gd is more far from Au in the figure 9 than in the figure 8. Gd being not covalent linked with the gold atom on figure 9.

In the present method, reduction of the salts is made in an aqueous solvent, salts of metal being chosen from a list comprising Gd, Eu,Tb, Ce, Mn, Fe, Zn, Ag, in the presence of a polymer, to obtain complexes, before obtaining nanoparticles realized after reduction. This method forms ionic bonds (i.e. electrostatic weak bonds) between the atom of gold and the atoms of metal in the created nanoparticle for the two mechanisms illustrated on figure 8 and on figure 9, whereas covalent bonds (strong bonds) are forms in prior art methods between the atom of gold and the atom of metal in view of the use of linkers or the like, for obtaining gold nanoparticles.

Interactions between AuCl₄⁻ ions and mixtures of polymers molecules (attractive ion-dipole interactions versus repulsive interactions due to hydrophobicity) are important in controlling the competition between the reactivity of AuCl₄⁻ reduction both in the bulk solution, which causes the nucleation of gold seeds.

The addiction of GdCl₃ to the gold-polymer complex induce a migration of Gd³⁺ ions inside polymer chains and consequent reduction to Gd²⁺.

Reduction with sodium borohydride NaBH₄ to obtain final nanoparticles

This result is likely due to the strong competition between PEG diacid and other polymers during reduction process of HAuCl₄

### Characterization of Gd-Au NPs

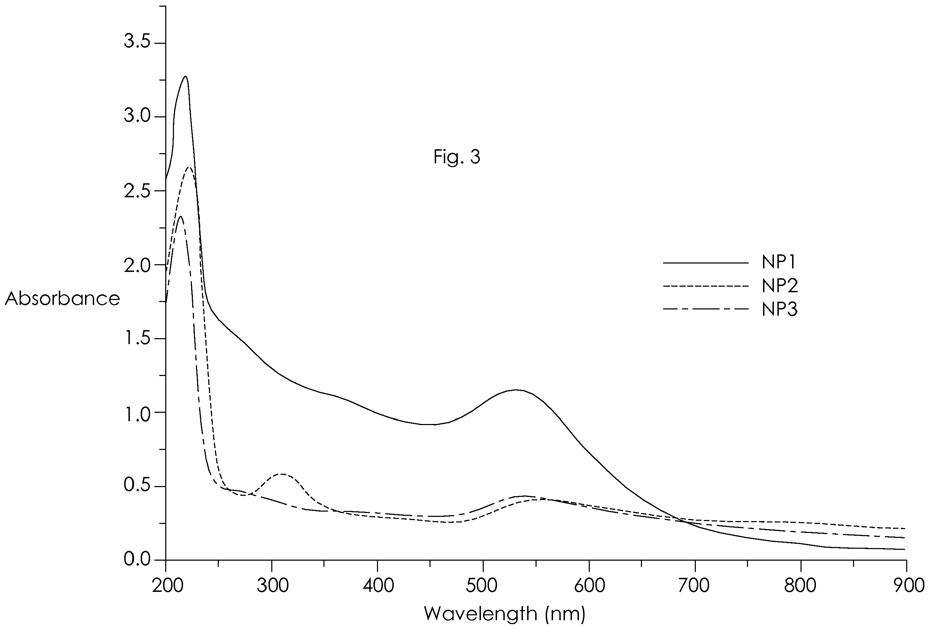

Figure 3 report absorption spectra of hybrid Gd- AuNPs, all characterized by a small peak at 300 nm and a surface plasmon band in the range 530-550 nm. The slow shift of the band position depends on the ratio of the gold salt and the capping materials during the reaction processes.

Gd-AuNP1 (Gd-Au-Col) shows a plasmon peak at 532 nm. This peak is generally ascribed to collective oscillation, known as the surface plasmon oscillation of the metal electrons in the conduction band, due to interaction of electrons with light of that wavelength. Col can be used as stabilizing polymers for AuNPs because of the dispersed solutions could be obtained due to the formation of coordination bands between Au and Gd ions with the amine or carboxylic groups respectively.

The absorption peak due at Gd-AuNP2 (Gd-Au-PEG-Col) is centered at 545 nm .This chelation evenly better dispersed Au ions and Gd which were reduced to form single AuNPs of relatively uniform size.

Gd-AuNP3 (Gd-Au-PEG) shows a strong resonance band at around 300 nm and a weaker one at 520 nm.

The shape of the nanomaterial can be modified depending on the concentrations of reagents and protocol used in the synthesis. As an example, NP1 obtained by the process of figure 8 shows bimetallic gold spheres with a diameter of around 50nm (see figure 1a), whereas NP3 obtained by the process of figure 8 shows snows shape particles of about 80nm (see figure 1c), possibly due to the presence of CHIT in the growth process.

The presence of Gd ions enhance the Raman signal of biomolecules, e.g. protein or natural polymer capped onto gold nanoparticles.

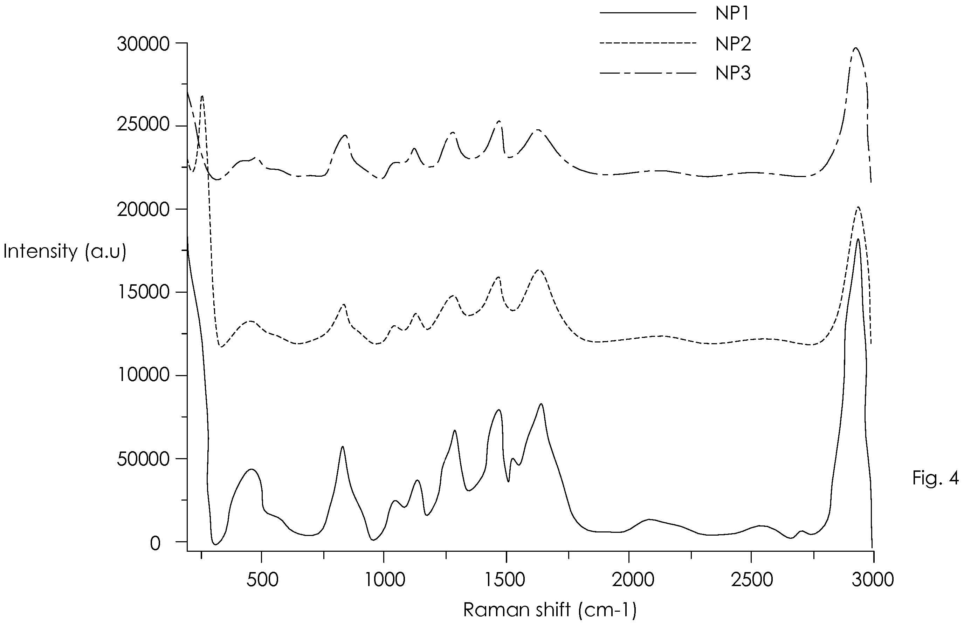

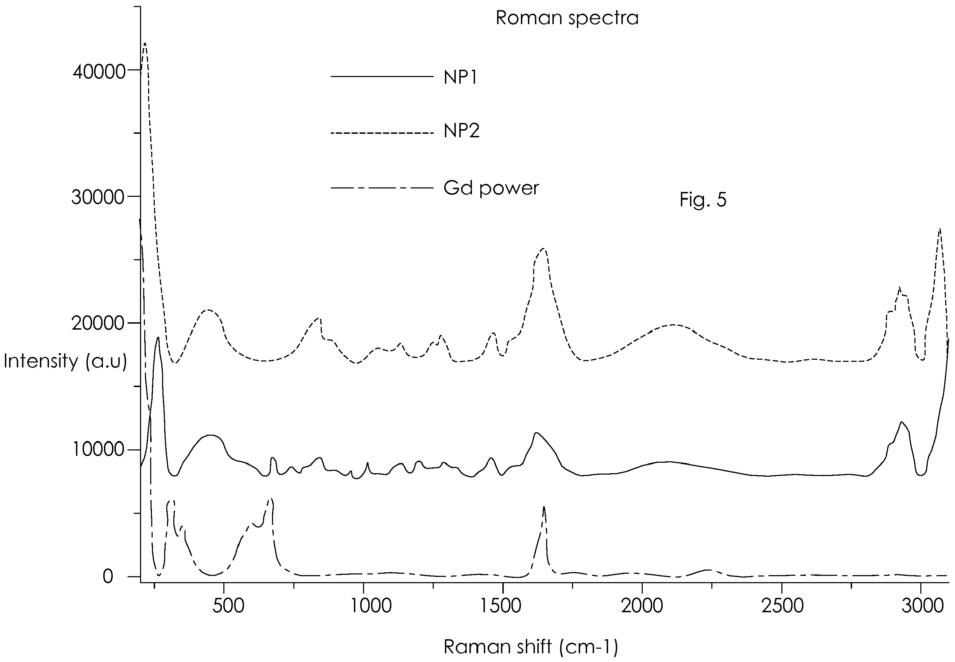

Raman spectra for powder GdCl₃ and GdAuNPs at 4.5 10⁻⁴ M are show in figures 4 and 5. The region from 1200 to 1550 cm⁻¹ corresponds to vibrations of N-H bending and C-N stretching, while the region between 1550 and 1750 cm⁻¹ corresponds to the C=O stretching mode. The band observed near 836 cm⁻¹ corresponds to vibrations of the aromatic ring. This enhancement is the result of the electric field around the gold nanoparticles interacting with collagen molecules in the presence of gadolinium. The vibration mode for the amine group is located at 1630 cm⁻¹, this band showing a signal enhancement factor of around 40 times higher than that of free collagen. This increment in the Raman signal of collagen could be due to a combination of three factors:
- formation of well-defined hot spots where Gd²⁺ ions work as spacers between gold nanoparticles
- the refractive index contrast produced by the presence of Gd²⁺ ions causing an increment in the electric field around the nanoparticle and producing a higher enhancement factor
- a charge transfer effect between gold nanoparticles and Gd²⁺ ions.

The invention provides inter alia synthesis of Gd-AuNPs in an aqueous solvent by reducing tetraclororoauric acid (HAuCl₄) in the presence of GdCl₃ and different polymers (PEG-diacid, Col, ALG, CHIT) using sodium borohydride (NaBH₄) as a reducing agent by using two protocols. Particles formation and growth were controlled by the amphiphilic (dual nature) character of the polymers. Synthesis are easy and brief.

The invention provides nanomaterial product that can be used as a substrate for Enhanced Raman Spectroscopy, or as a contrast agent for Magnetic Resonance Imaging, or as a therapeutic agent, a radio sensitive agent, a thermo-therapy agent, a radioactive agent, or a phototherapy agent.

The nanomaterial product has a low toxicity. Biopolymers such as PEG, Col, CHIT, ALG are used as stabilizer agent during the production of the nanomaterial and also as support for fixing biomolecules (e.g. DNA, medical product), enabling the use of the nanomaterial for the delivery of drugs for cancer treatment, by passively and actively targeting to tumor cells.

The method presents very fast steps to obtain nanoparticles.

## Claims

1. A method for producing nanomaterial product which comprises at least one nanoparticle gold-metal-polymer, the polymer comprising at least one biopolymer, the metal being chosen among: Gd, Co, Eu, Tb, Ce, Mn, Fe, Zn, Ag,
- the method being realized in an aqueous solvent,
- and presenting a step of reducing gold ions and metal ions by introducing a reducing agent, in the presence of the biopolymer, the biopolymer being used as a stabilizer agent.

2. A method according to claim 1, wherein the method being realized without chemical surfactant and without chemical linker.

3. A method according to claims 1 or 2, wherein the reduction being realized in the presence of a biopolymer mixture which comprises at least two biopolymers, one of the biopolymer comprising COOH group.

4. A method according to one of the claims 1-3, wherein the method comprises the successive steps:
- (Ai) introduction of metal ions in the aqueous solvent which comprises AuCl₄⁻, for realizing complexation between metal ions and gold ions conducted to form gold-metal complex, during a first period;
- (Aii) stacking, by mixing said gold-metal complex with a first biopolymer introduced in the aqueous solvent, for obtaining a gold-metal-polymer complex, during a second period;
- (Aiii) reduction of metal ions and gold ions of the gold-metal-polymer complex, by introducing the reducing agent, during a third period.

5. A method according to one of the claims 1-3, wherein the method comprises the successive steps:
- (Bi) staking by mixing tetrachloroauric acid HAuCl₄ with a first biopolymer for obtaining a gold polymer complex, during a first period,
- (Bii) complexation by addition of metal ions to the gold polymer complex during a second period, for obtaining a gold-polymer complex,
- (Biii) reduction of metal ions and gold ions of the gold-metal-polymer complex, by introducing a reducing agent, during a third period.

6. A method according to claims 4 or 5, wherein the method comprises a step of adding a second biopolymer: between the step (Aii) and the step (Aiii), or between the step (Bi) and the step (Bii).

7. A method according to claims 1 to 6, wherein the steps of the method are conducted at room temperature.

8. A method according to claims 4 to 7, wherein the first period, the second period and the third period last each less than fifteen minutes.

9. A method according to claims 1 to 8, wherein the first biopolymer and the second biopolymer are chosen among: collagen, chitosan, polyethylene glycol diacide, alginate.

10. Nanomaterial product synthetized as described in claims 1 to 9, with at least one nanoparticle gold-metal-polymer, the metal is chosen among: Gd, Co, Eu, Tb, Ce, Mn, Fe, Zn, Ag, the polymer being at least one biopolymer comprising COOH group.

11. Nanomaterial product synthetized as described in claim 10, with at least one nanoparticle gold-metal-polymers, the metal is chosen among: Gd, Co, Eu, Tb, Ce, Mn, Fe, Zn, Ag, the polymers being at least two biopolymers, one of the biopolymer comprising COOH group.

12. Nanomaterial product according to one of the previous claims 10-11, wherein the nanoparticle comprises:
- an inner core with one atom of gold and atoms of metal surrounded the atom of gold, and
- an external shell with the biopolymer(s).

13. Nanomaterial product according to one of the previous claims 10-11, wherein the nanoparticle comprises:
- an inner core with one atom of gold, and
- an external shell with atoms of metal and the biopolymer(s).

14. Nanomaterial product according to claims 10 to 13, wherein the biopolymer is chosen among: collagen, chitosan, polyethylene glycol diacide, alginate.

15. Nanomaterial product according to claims 10 to 14, wherein the metal is Gd, and the biopolymer is collagen.

16. Nanomaterial product according to claims 11 to 15, wherein the metal is Gd, and the biopolymers are collagen and PEG.

17. A nanomaterial product synthetized according to a method of claims 1-9, with at least one nanoparticle gold-metal-polymer, the metal being chosen among Gd, Co, Eu, Tb, Ce, Mn, Fe, Zn, Ag, the polymer being at least one biopolymer, ionic bonds being formed between the atom of gold and the atoms of metal.

18. Use of the nanomaterial product of claims 10 to 17, as
- a substrate for Enhanced Raman Spectroscopy;
- a contrast agent for Magnetic Resonance Imaging;
- a therapeutic agent;
- a radio sensitive agent;
- a thermo-therapy agent;
- a radioactive agent;
- a phototherapy agent.
